# FASCICULE DE BREVET EUROPEEN

(11) **EP 1 493 426 B1**
(45) Date de publication et mention de la délivrance du brevet: **12.08.2009**
(21) Numéro de dépôt: 04291547.0
(22) Date de dépôt: 18.06.2004
(51) Int. Cl.: A61Q 5/10, A61K 8/41, A61K 8/49

(54) **Composition tinctoriale comprenant au moins une base d'oxydation, le 2-chloro 6-méthyl 3-amino phénol et le 3-méthyl 1-phényl 5-pyrazolone**
Färbemittel enthaltend mindestens einen Entwickler, 2-Chlor-6-methyl-3-aminophenol und 3-Methyl-1-phenyl-5-pyrazolon
Dyeing composition comprising a developer, 2-chloro-6-methyl-3-aminophenol and 3-methyl-1-phenyl-5-pyrazolone

(30) Priorité: 01.07.2003 FR 0307957
(43) Date de publication de la demande: 05.01.2005
(73) Titulaire: L'ORÉAL, 75008 Paris (FR)
(72) Inventeur: Audousset, Marie-Pascale, 92600 Asnières (FR); Bone, Eric, 92500 Rueil Malmaison (FR)
(74) Mandataire: Fevrier, Murielle Françoise E.

(56) Documents cités:
- EP-A- 0 039 030
- DE-A- 4 440 955
- US-B1- 6 284 003

## Description

L'invention a pour objet une composition pour la teinture d'oxydation des fibres kératiniques, et en particulier des fibres kératiniques humaines telles que les cheveux, comprenant au moins une base d'oxydation, le 2-chloro 6-méthyl 3-amino phénol et le 3-méthyl 1-phényl 5-pyrazolone.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il a déjà été proposé, notamment dans la demande de brevet allemand DE 30 16 008, des compositions pour la teinture d'oxydation des fibres kératiniques comprenant du 2-chloro 6-méthyl 3-amino phénol ou du 2-méthyl 6-chloro 3-amino phénol à titre de coupleur, en association avec des bases d'oxydation classiquement utilisées pour la teinture d'oxydation, telles que par exemple certaines para-phénylènediamines, du para-aminophénol ou des bases hétérocycliques.

La demande de brevet WO 96/15765 décrit des compositions pour la teinture d'oxydation des fibres kératiniques comprenant le 2-chloro 6-méthyl 3-amino phénol à titre de coupleur et le 2-(2,5-diaminophényl) éthanol à titre de base d'oxydation.

La demande de brevet WO 96/15766 décrit des compositions pour la teinture d'oxydation des fibres kératiniques comprenant l'association spécifique du 2-chloro 6-méthyl 3-amino phénol à titre de coupleur et d'une base d'oxydation particulière choisie parmi les dérivés de para-aminophénol substitué en position 2 ou 3 comme par exemple le 3-méthyl 4-amino phénol, le 2-allyl 4-amino phénol ou bien encore le 2-aminométhyl 4-amino phénol.

Le brevet FR 1 426 889 décrit des compositions pour la teinture d'oxydation des fibres kératiniques comprenant du 3-méthyl 1-phényl 5-pyrazolone à titre de coupleur, en association avec une para-phénylènediamine particulière à titre de base d'oxydation.

De telles compositions ne sont cependant pas entièrement satisfaisantes notamment du point de vue de la tenue des colorations obtenues vis-à-vis des diverses agressions que peuvent subir les cheveux, et en particulier vis-à-vis des shampooings, de la lumière, de la sueur et des déformations permanentes et du point de vue de la puissance, de la sélectivité et de la chromaticité des colorations obtenues. De plus, lorsqu'il est présent dans la composition tinctoriale, le 2-chloro 6-méthyl 3-amino phénol n'est pas toujours très stable, ce qui a pour effet l'obtention de colorations non satisfaisantes.

Le but de la présente invention est de fournir de nouvelles compositions pour la teinture d'oxydation des fibres kératiniques ne présentant pas les inconvénients de celles de l'art antérieur. En particulier, le but de la présente invention est de fournir de nouvelles compositions conduisant à des colorations aux nuances variées, puissantes, chromatiques, esthétiques, peu sélectives et résistant bien aux diverses agressions que peuvent subir les cheveux tels que les shampooings, la lumière, la sueur et les déformations permanentes.

Ce but est atteint avec la présente invention qui a pour objet une composition pour la teinture d'oxydation des fibres kératiniques comprenant, dans un milieu de teinture approprié :
- au moins une base d'oxydation ;
- au moins un coupleur choisi parmi le 2-chloro 6-méthyl 3-amino phénol et ses sels d'addition ; et
- le 3-méthyl 1-phényl 5-pyrazolone et / ou l'un de ses sels d'addition.

La composition de la présente invention permet en particulier d'obtenir une coloration des fibres kératiniques puissante, chromatique, esthétique, peu sélective et plus résistante aux différentes agressions que peuvent subir les fibres kératiniques. De plus, le 2-chloro 6-méthyl 3-amino phénol présente une bonne stabilité dans la composition de l'invention.

L'invention a aussi pour objet un procédé de teinture d'oxydation des fibres kératiniques, et en particulier des fibres kératiniques humaines telles que les cheveux, mettant en oeuvre cette composition.

Un autre objet de l'invention est l'utilisation de la composition de la présente invention pour la teinture d'oxydation des fibres kératiniques, et en particulier des fibres kératiniques humaines telles que les cheveux.

La présente invention a également pour objet l'utilisation du 3-méthyl 1-phényl 5-pyrazolone pour la stabilisation du 2-chloro 6-méthyl 3-amino phénol dans les compositions.

Les bases d'oxydation pouvant être utilisées dans la composition tinctoriale conforme à l'invention sont de préférence choisies parmi les para-phénylènediamines, les bases doubles, les para-aminophénols, les ortho-aminophénols, les bases d'oxydation hétérocycliques et leurs sels d'addition.

Parmi les para-phénylènediamines utilisables à titre de base d'oxydation dans la composition tinctoriale conforme à l'invention, on peut notamment citer les composés de formule (I) suivante et leurs sels d'addition : 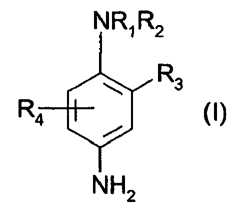 dans laquelle :
- R₁ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ; un radical alkyle en C₁-C₄ substitué par un groupement azoté ; un radical phényle ou un radical 4'-aminophényle ;
- R₂ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ou un radical alkyle en C₁-C₄ substitué par un groupement azoté ;
- R₁ et R₂ peuvent former ensemble, avec l'atome d'azote auquel ils sont rattachés, un hétérocycle comportant de 5 à 8 chaînons, éventuellement substitué par un ou plusieurs groupements choisis parmi les radicaux alkyle en C₁-C₄, amino, hydroxyalkyle en C₁-C₄ et trialkylammonium ;
- R₃ représente un atome d'hydrogène ; un atome d'halogène tel qu'un atome de chlore, de brome, d'iode ou de fluor ; un radical alkyle en C₁-C₄ ; un radical monohydroxyalkyle en C₁-C₄ ; un radical hydroxyalcoxy en C₁-C₄ ; un radical acétylaminoalcoxy en C₁-C₄ ; un radical mésylaminoalcoxy en C₁-C₄ ou un radical carbamoylaminoalcoxy en C₁-C₄ ;
- R₄ représente un atome d'hydrogène ; un atome d'halogène ou un radical alkyle en C₁-C₄.

Parmi les para-phénylènediamines de formule (I) ci-dessus, on peut plus particulièrement citer la para-phénylènediamine ; la para-toluylènediamine ; la 2-chloro para-phénylènediamine ; la 2,3-diméthyl para-phénylènediamine ; la 2,6-diméthyl para-phénylènediamine ; la 2,6-diéthyl para-phénylènediamine ; la 2,5-diméthyl para-phénylènediamine ; la N,N-diméthyl para-phénylènediamine ; la N,N-diéthyl para-phénylènediamine ; la N,N-dipropyl para-phénylènediamine ; la 4-amino N,N-diéthyl 3-méthyl aniline ; la N,N-bis-(β-hydroxyéthyl) para-phénylènediamine ; la 4-N,N-bis-(β-hydroxyéthyl)amino 2-méthyl aniline ; la 4-N,N-bis-(β-hydroxyéthyl)amino 2-chloro aniline ; la 2-(β-hydroxyéthyl) para-phénylènediamine ; la 2-fluoro para-phénylènediamine ; la 2-isopropyl para-phénylènediamine ; la N-(β-hydroxypropyl) para-phénylènediamine ; la 2-hydroxyméthyl para-phénylènediamine ; la N,N-diméthyl 3-méthyl para-phénylènediamine ; la N,N-(éthyl, β-hydroxyéthyl) para-phénylènediamine ; la N-(β,γ-dihydroxypropyl) para-phénylènediamine ; la N-(4'-aminophényl) para-phénylènediamine ; la N-phényl para-phénylènediamine ; la 2-(β-hydroxyéthyloxy) para-phénylènediamine ; la 2-(β-acétylaminoéthyloxy) para-phénylènediamine ; la N-(β-méthoxyéthyl) para-phénylènediamine ; le 2-(β-hydroxyéthyl)amino 5-amino toluène et leurs sels d'addition.

Parmi les para-phénylènediamines de formule (I) ci-dessus, on préfère tout particulièrement la para-phénylènediamine ; la para-toluylènediamine ; la 2-isopropyl para-phénylènediamine ; la 2-(β-hydroxyéthyl) para-phénylènediamine ; la 2-(β-hydroxyéthyloxy) para-phénylènediamine ; la 2,6-diméthyl para-phénylènediamine ; la 2,6-diéthyl para-phénylènediamine ; la 2,3-diméthyl para-phénylènediamine ; la N,N-bis-(β-hydroxyéthyl) para-phénylènediamine ; la 2-chloro para-phénylènediamine ; la 2-(β-acétylaminoéthyloxy) para-phénylènediamine et leurs sels d'addition.

Selon l'invention, on entend par bases doubles les composés comportant au moins deux noyaux aromatiques sur lesquels sont portés des groupements amino et / ou hydroxyle.

Parmi les bases doubles utilisables à titre de base d'oxydation dans la composition tinctoriale conforme à l'invention, on peut notamment citer les composés répondant à la formule (II) suivante, et leurs sels d'addition : 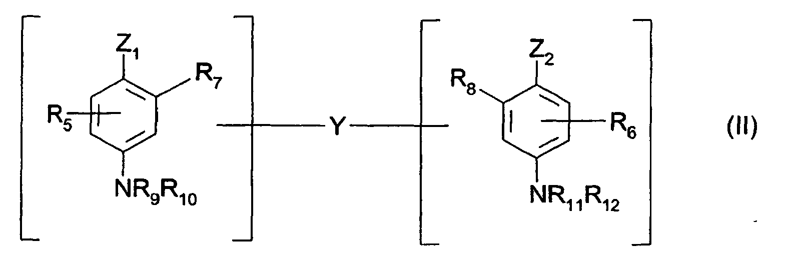 dans laquelle :
- Z₁ et Z₂, identiques ou différents, représentent un radical hydroxyle ou amino pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et / ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ; un atome d'halogène ; un radical alkyle en C₁-C₄ ; un radical monohydroxyalkyle en C₁-C₄ ; un radical polyhydroxyalkyle en C₂-C₄ ; un radical aminoalkyle en C₁-C₄ ou un bras de liaison Y ;
- R₇, R₈, R₉, R₁₀, R₁₁ et R₁₂, identiques ou différents, représentent un atome d'hydrogène ; un bras de liaison Y ; un radical alkyle en C₁-C₄ ou un radical monohydroxyalkyle en C₁-C₄ ;
étant entendu que les composés de formule (II) ne comportent qu'un seul bras de liaison Y par molécule.

Parmi les bases doubles de formule (II) ci-dessus dérivées de la para-phénylènediamine, on peut plus particulièrement citer le N,N'-bis-(β-hydroxyéthyl) N,N'-bis-(4'-aminophényl) 1,3-diamino propanol ; la N,N'-bis-(β-hydroxyéthyl) N,N'-bis-(4'-aminophényl) éthylènediamine ; la N,N'-bis-(4'-aminophényl) tétraméthylènediamine ; la N,N'-bis-(β-hydroxyéthyl) N,N'-bis-(4'-aminophényl) tétraméthylènediamine ; la N,N'-bis-(4'-méthyl-aminophényl) tétraméthylènediamine ; la N,N'-bis-(éthyl) N,N'-bis-(4'-amino, 3'-méthylphényl) éthylènediamine ; le 1,8-bis-(2',5'-diamino phénoxy)-3,6-dioxaoctane et leurs sels d'addition.

Parmi les bases doubles de formule (II) ci-dessus dérivées du para-aminophénol, on peut plus particulièrement citer le 4-amino 6-[(5'-amino 2'-hydroxy 3'-méthyl phényl)méthyl] 2-méthyl phénol ; le bis-(5'-amino 2'-hydroxy phényl) méthane et leurs sels d'addition.

Parmi ces bases doubles de formule (II), le N,N'-bis-(β-hydroxyéthyl) N,N'-bis-(4'-aminophényl) 1,3-diamino propanol ; le 1,8-bis-(2,5-diaminophénoxy)-3,5-dioxaoctane et leurs sels d'addition sont particulièrement préférés.

Parmi les para-aminophénols utilisables à titre de bases d'oxydation dans la composition tinctoriale conforme à l'invention, on peut notamment citer les composés répondant à la formule (III) suivante, et leurs sels d'addition : 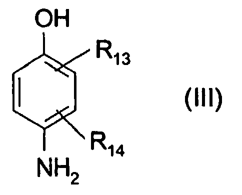 dans laquelle :
- R₁₃ représente un atome d'hydrogène ; un atome d'halogène ; un radical alkyle en C₁-C₄ ; un radical monohydroxyalkyle en C₁-C₄ ; un radical alcoxy(C₁-C₄)alkyle(C₁-C₄) ; un radical aminoalkyle en C₁-C₄ ou un radical hydroxyalkyl(C₁-C₄)aminoalkyle en C₁-C₄ ;
- R₁₄ représente un atome d'hydrogène ; un atome d'halogène ; un radical alkyle en C₁-C₄ ; un radical monohydroxyalkyle en C₁-C₄ ; un radical polyhydroxyalkyle en C₂-C₄ ; un radical aminoalkyle en C₁-C₄ ; un radical cyanoalkyle en C₁-C₄ ou un radical alcoxy(C₁-C₄)alkyle(C₁-C₄) ;
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol ; le 4-amino 3-méthyl phénol ; le 4-amino 3-fluoro phénol ; le 4-amino 3-hydroxyméthyl phénol ; le 4-amino 2-méthyl phénol ; le 4-amino 2-hydroxyméthyl phénol ; le 4-amino 2-méthoxyméthyl phénol ; le 4-amino 2-aminométhyl phénol ; le 4-amino 2-(β-hydroxyéthyl aminométhyl) phénol ; le 4-amino 2-fluoro phénol ; le 4-amino 2-chloro phénol ; le 4-amino 2,6-dichloro phénol et leurs sels d'addition.

Parmi les ortho-aminophénols utilisables à titre de base d'oxydation dans la composition tinctoriale conforme à l'invention, on peut plus particulièrement citer le 2-amino phénol ; le 2-amino 5-méthyl phénol ; le 2-amino 6-méthyl phénol ; le 5-acétamido 2-amino phénol et leurs sels d'addition.

Parmi les bases hétérocycliques utilisables à titre de base d'oxydation dans la composition tinctoriale conforme à l'invention, on peut plus particulièrement citer les dérivés pyridiniques, les dérivés pyrimidiniques, les dérivés pyrazoliques, les dérivés pyrazolo-pyrimidiniques et leurs sels d'addition.

Parmi les dérivés pyridiniques, on peut plus particulièrement citer les composés décrits par exemple dans les brevets GB 1 026 978 et GB 1 153 196, comme la 2,5-diamino pyridine ; la 2-(4-méthoxyphényl)amino 3-amino pyridine ; la 2,3-diamino 6-méthoxy pyridine ; la 2-(β-méthoxyéthyl)amino 3-amino 6-méthoxy pyridine ; la 3,4-diamino pyridine et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ainsi que leurs sels d'addition.

Parmi les dérivés pyrimidiniques, on peut plus particulièrement citer les composés décrits par exemple dans les brevets DE 23 59 399, JP 88-169571, JP 05-63124, EP 0 770 375 ou la demande de brevet WO 96/15765 comme la 2,4,5,6-tétra-aminopyrimidine ; la 4-hydroxy 2,5,6-triaminopyrimidine ; la 2-hydroxy 4,5,6-triaminopyrimidine ; la 2,4-dihydroxy 5,6-diaminopyrimidine ; la 2,5,6-triaminopyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 38 43 892, DE 41 33 957 et les demandes de brevet WO 94/08969, WO 94/08970, FR-A-2 733 749 et DE 195 43 988 comme le 4,5-diamino 1-méthyl pyrazole ; le 4,5-diamino 1-(β-hydroxyéthyl) pyrazole ; le 3,4-diamino pyrazole ; le 4,5-diamino 1-(4'-chlorobenzyl) pyrazole ; le 4,5-diamino 1,3-diméthyl pyrazole ; le 4,5-diamino 3-méthyl 1-phényl pyrazole ; le 4,5-diamino 1-méthyl 3-phényl pyrazole ; le 4-amino 1,3-diméthyl 5-hydrazino pyrazole ; le 1-benzyl 4,5-diamino 3-méthyl pyrazole ; le 4,5-diamino 3-tert-butyl 1-méthyl pyrazole ; le 4,5-diamino 1-(β-hydroxyéthyl) 3-méthyl pyrazole ; le 4,5-diamino 1-tert-butyl 3-méthyl pyrazole ; le 4,5-diamino 1-éthyl 3-méthyl pyrazole ; le 4,5-diamino 1-éthyl 3-(4'-méthoxyphényl) pyrazole ; le 4,5-diamino 1-éthyl 3-hydroxyméthyl pyrazole ; le 4,5-diamino 3-hydroxyméthyl 1-méthyl pyrazole ; le 4,5-diamino 3-hydroxyméthyl 1-isopropyl pyrazole ; le 4,5-diamino 3-méthyl 1-isopropyl pyrazole ; le 4-amino 5-(2'-aminoéthyl)amino 1,3-diméthyl pyrazole ; le 3,4,5-triamino pyrazole ; le 1-méthyl 3,4,5-triamino pyrazole ; le 3,5-diamino 1-méthyl 4-méthylamino pyrazole ; le 3,5-diamino 4-(β-hydroxyéthyl)amino 1-méthyl pyrazole et leurs sels d'addition.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 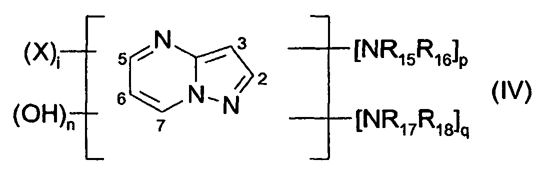 dans laquelle :
- R₁₅, R₁₆, R₁₇ et R₁₈, identiques ou différents, désignent un atome d'hydrogène ; un radical alkyle en C₁-C₄ ; un radical aryle ; un radical hydroxyalkyle en C₁-C₄ ; un radical polyhydroxyalkyle en C₂-C₄ ; un radical (C₁-C₄)alcoxy alkyle en C₁-C₄ ; un radical aminoalkyle en C₁-C₄, l'amine pouvant être protégée par un radical acétyle, uréido ou sulfonyle ; un radical (C₁-C₄)alkylamino alkyle en C₁-C₄ ; un radical di-[(C₁-C₄)alkyl] amino alkyle en C₁-C₄, les radicaux dialkyles pouvant former un cycle carboné ou un hétérocycle à 5 ou 6 chaînons ; un radical hydroxy(C₁-C₄)alkyl- ou di-[hydroxy(C₁-C₄) alkyl]-amino alkyle en C₁-C₄ ;
- les radicaux X, identiques ou différents, désignent un atome d'hydrogène ; un radical alkyle en C₁-C₄ ; un radical aryle ; un radical hydroxyalkyle en C₁-C₄ ; un radical polyhydroxyalkyle en C₂-C₄ ; un radical amino alkyle en C₁-C₄ ; un radical (C₁-C₄)alkyl amino alkyle en C₁-C₄ ; un radical di-[(C₁-C₄)alkyl] amino alkyle en C₁-C₄, les dialkyles pouvant former un cycle carboné ou un hétérocycle à 5 ou 6 chaînons ; un radical hydroxy(C₁-C₄)alkyl ou di-[hydroxy(C₁-C₄ )alkyl]amino alkyle en C₁-C₄ ; un radical amino ; un radical (C₁-C₄)alkyl- ou di-[(C₁-C₄)alkyl]-amino ; un atome d'halogène ; un groupe acide carboxylique ; un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7).

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

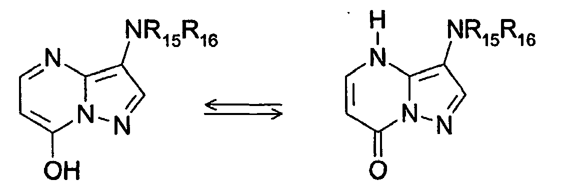

Parmi les pyrazolo-[1,5-a]-pyrimidines de formule (IV) ci-dessus on peut notamment citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-Amino-pyrazolo[1,5-a]pyrimidin-7-yl)-(2-hydroxy-éthyl)-amino]-éthanol ; le 2-[(7-Amino-pyrazolo[1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY ;
- R. Vishdu, H. Navedul, Indian J. Chem., 34b (6), 514, 1995 ;
- N.S. Ibrahim, K.U. Sadek, F.A. Abdel-Al, Arch. Pharm., 320, 240, 1987 ;
- R.H. Springer, M.B. Scholten, D.E. O'Brien, T. Novinson, J.P. Miller, R.K. ; Robins, J. Med. Chem., 25, 235, 1982 ;
- T. Novinson, R.K. Robins, T.R. Matthews, J. Med. Chem., 20, 296, 1977 ;
- US 3907799 ICN PHARMACEUTICALS.

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977 ;
- E. Alcade, J. De Mendoza, J.M. Marcia-Marquina, C. Almera, J. Elguero, J. Heterocyclic Chem., 11 (3), 423, 1974;
- K. Saito, I. Hori, M. Higarashi, H. Midorikawa, Bull. Chem. Soc. Japan, 47(2), 476, 1974.

La composition selon l'invention peut en outre contenir un ou plusieurs coupleurs additionnels choisis parmi les coupleurs conventionnellement utilisés pour la teinture d'oxydation des fibres kératiniques. Parmi ces coupleurs, on peut notamment citer les méta-phénylènediamines, les méta-aminophénols différents du 2-chloro 6-méthyl 3-amino phénol, les méta-diphénols, les coupleurs naphtaléniques, les coupleurs hétérocycliques différents du 3-méthyl 1-phényl 5-pyrazolone ainsi que leurs sels d'addition.

A titre d'exemple, on peut citer le 2-méthyl 5-amino phénol ; le 5-N-(β-hydroxyéthyl)amino 2-méthyl phénol ; le 3-amino phénol ; le 1,3-dihydroxy benzène ; le 1,3-dihydroxy 2-méthyl benzène ; le 4-chloro 1,3-dihydroxy benzène ; le 2,4-diamino 1-(β-hydroxyéthyloxy) benzène ; le 2-amino 4-(β-hydroxyéthylamino) 1-méthoxy benzène ; le 1,3-diamino benzène ; le 1,3-bis-(2,4-diaminophénoxy) propane ; la 3-uréido aniline ; le 3-uréido 1-diméthylamino benzène ; le sésamol ; le 1-β-hydroxyéthylamino-3,4-méthylènedioxy benzène ; l'α-naphtol ; le 2 méthyl-1-naphtol ; le 6-hydroxy indole ; le 4-hydroxy indole ; le 4-hydroxy N-méthyl indole ; la 2-amino-3-hydroxy pyridine ; la 6- hydroxy benzomorpholine ; la 3,5-diamino-2,6-diméthoxy pyridine ; le 1-N-(β-hydroxyéthyl)amino-3,4-méthylènedioxy benzène ; le 2,6-bis-(β-hydroxyéthylamino) toluène et leurs sels d'addition.

La ou les bases d'oxydation présentes dans la composition de l'invention sont en général présentes chacune en quantité comprise entre 0,001 à 10 % en poids environ du poids total de la composition tinctoriale, de préférence entre 0,005 et 6 %.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

Le 3-méthyl 1-phényl 5-pyrazolone et / ou ses sels d'addition sont en général présents en quantité comprise entre 0,001 et 10 % en poids environ du poids total de la composition tinctoriale, de préférence entre 0,005 et 6 %.

De préférence, le rapport entre le poids du 2-chloro 6-méthyl 3-amino phénol et / ou de ses sels d'addition et le poids du 3-méthyl 1-phényl 5-pyrazolone et / ou de ses sels d'addition est compris entre 1 et 100.

D'une manière générale, les sels d'addition des composés utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre comprendre un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions comprises entre 1 et 40 % en poids environ par rapport au poids total de la composition tinctoriale, et de préférence entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques autres que les polymères associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus peuvent être présents en quantité comprise pour chacun d'eux entre 0,001 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XVI) suivante : 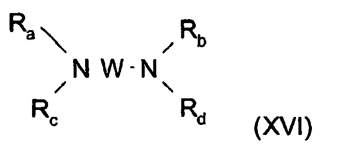 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel la composition conforme à l'invention telle que définie précédemment est appliquée sur les fibres kératiniques, et la couleur est révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le comprenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale conforme à l'invention et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

La présente invention a également pour objet l'utilisation du 3-méthyl 1-phényl 5-pyrazolone et / ou de ses sels d'addition pour la stabilisation du 2-chloro 6-méthyl 3-amino phénol et / ou de ses sels d'addition en solution.

Les exemples qui suivent servent à illustrer l'invention sans toutefois présenter un caractère limitatif.

### EXEMPLES

Les compositions tinctoriales ci-dessous sont préparées :

| **Exemple** | **1** | **2** | **3** | **4** |
|---|---|---|---|---|
| 2-chloro 6-méthyl 3-amino phénol | 1,26 g | 1,26 g | 1,26 g | 1,26 g |
| Para-aminophénol | 0,872 g | | | |
| 3-méthyl 4-amino phénol | | | | 0,984 g |
| Para-phénylènediamine | | | 0,864 g | |
| Para-toluènediamine | | 0,976 g | | |
| 3-méthyl 1-phényl 5-pyrazolone | 0,15 g | 0,15 g | 0,15 g | 0,15 g |
| Support de teinture | (*) | (*) | (*) | (*) |
| Eau déminéralisée q.s.p. | 100 g | 100 g | 100 g | 100 g |

| | | | | |
|---|---|---|---|---|
| (*) : support de teinture commun Alcool oléique polyglycérolé à 2 moles de glycérol 4 g Alcool oléique polyglycérolé à 4 moles de glycérol (78 % M.A.) 5,69 g M.A. Acide oléique 3,0 g Amine oléique à 2 moles d'oxyde d'éthylène vendue sous la dénomination commerciale ETHOMEEN O12 par la société AKZO 7 g Laurylamino succinamate de diéthylaminopropyle, sel de sodium à 55 % M.A. 3,0 g M.A. Alcool oléique 5,0 g Diéthanolamide d'acide oléique 12,0 g Propylène glycol 3,5 g Alcool éthylique 7,0 g Dipropylène glycol 0,5 g Monométhyléther de propylène glycol 9 g Métabisulfite de sodium en solution aqueuse à 35 % M.A. 0,455 g M.A. Acétate d'ammonium 0,8 g Antioxydant, séquestrant q.s. Parfum, conservateur q.s. Ammoniaque à 20 % de NH₃ 10 g | | | | |

La composition tinctoriale est mélangée extemporanément avec une fois son poids d'eau oxygénée à 20 volumes.

Le mélange ainsi réalisé est appliqué sur des mèches de cheveux gris à 90% de blancs naturels, à raison de 28 g pour 3 g de cheveux, pendant 30 minutes. Les cheveux sont ensuite rincés, lavés avec un shampooing standard et séchés.

La coloration capillaire est évaluée de manière visuelle. Les nuances obtenues figurent dans le tableau ci-dessous.

| **Exemple** | **1** | **2** | **3** | **4** |
|---|---|---|---|---|
| Hauteur de ton | Blond | Blond foncé | Blond foncé | Blond |
| Reflet | Rouge cuivré | Violacé | Violine | Rouge |

## Revendications

1. Composition pour la teinture d'oxydation des fibres kératiniques comprenant, dans un milieu de teinture approprié :
- au moins une base d'oxydation ;
- au moins un coupleur choisi parmi le 2-chloro 6-méthyl 3-amino phénol et ses sels d'addition ; et
- le 3-méthyl 1-phénol 5-pyrazolone et / ou l'un de ses sels d'addition.

2. Composition selon la revendication 1, dans laquelle la ou les bases d'oxydation sont choisies parmi les para-phénylènediamines, les bases doubles, les para-aminophénols, les ortho-aminophénols, les bases d'oxydation hétérocycliques ainsi que leurs sels d'addition.

3. Composition selon la revendication 2, dans laquelle les para-phénylènediamines sont choisies parmi les composés de formule (I) suivante et leurs sels d'addition : 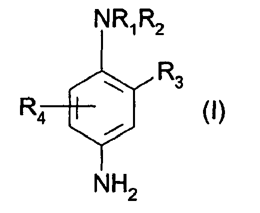 dans laquelle :
- R₁ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ; un radical alkyle en C₁-C₄ substitué par un groupement azoté ; un radical phényle ou un radical 4'-aminophényle ;
- R₂ représente un atome d'hydrogène ; un radical alkyle en C₁-C₄ ; un radical monohydroxyalkyle en C₁-C₄ ; un radical polyhydroxyalkyle en C₂-C₄ ; un radical alcoxy(C₁-C₄)alkyle(C₁-C₄) ou un radical alkyle en C₁-C₄ substitué par un groupement azoté ;
- R₁ et R₂ peuvent former ensemble, avec l'atome d'azote auquel ils sont rattachés, un hétérocycle comportant de 5 à 8 chaînons, éventuellement substitué par un ou plusieurs groupements choisis parmi les radicaux alkyle en C₁-C₄, amino, hydroxyalkyle en C₁-C₄ et trialkylammonium ;
- R₃ représente un atome d'hydrogène ; un atome d'halogène tel qu'un atome de chlore, de brome, d'iode ou de fluor ; un radical alkyle en C₁-C₄ ; un radical monohydroxyalkyle en C₁-C₄ ; un radical hydroxyalcoxy en C₁-C₄ ; un radical acétylaminoalcoxy en C₁-C₄ ; un radical mésylaminoalcoxy en C₁-C₄ ou un radical carbamoylaminoalcoxy en C₁-C₄ ;
- R₄ représente un atome d'hydrogène ; un atome d'halogène ou un radical alkyle en C₁-C₄.

4. Composition selon la revendication 3, dans laquelle les para-phénylènediamines de formule (I) sont choisies parmi la para-phénylènediamine ; la para-toluylènediamine ; la 2-chloro para-phénylènediamine ; la 2,3-diméthyl para-phénylènediamine ; la 2,6-diméthyl para-phénylènediamine ; la 2,6-diéthyl para-phénylènediamine ; la 2,5-diméthyl para-phénylènediamine ; la N,N-diméthyl para-phénylènediamine ; la N,N-diéthyl para-phénylènediamine ; la N,N-dipropyl para-phénylènediamine ; la 4-amino N,N-diéthyl 3-méthyl aniline ; la N,N-bis-(β-hydroxyéthyl) para-phénylènediamine ; la 4-N,N-bis-(β-hydroxyéthyl)amino 2-méthyl aniline - la 4-N,N-bis-(β-hydroxyéthyl)amino 2-chloro aniline ; la 2-(β-hydroxyéthyl) para-phénylènediamine ; la 2-fluoro para-phénylènediamine ; la 2-isopropyl para-phénylènediamine ; la N-(β-hydroxypropyl) para-phénylènediamine ; la 2-hydroxyméthyl para-phénylènediamine ; la N,N-diméthyl 3-méthyl para-phénylènediamine ; la N,N-(éthyl, β-hydroxyéthyl) para-phénylènediamine ; la N-(β,γ-dihydroxypropyl) para-phénylènediamine ; la N-(4'-aminophényl) para-phénylènediamine ; la N-phényl para-phénylènediamine ; la 2-(β-hydroxyéthyloxy) para-phénylènediamine ; la 2-(β-acétylaminoéthyloxy) para-phénylènediamine ; la N-(β-méthoxyéthyl) para-phénylènediamine et leurs sels d'addition.

5. Composition selon l'une quelconque des revendications 2 à 4, dans laquelle les bases doubles sont choisies parmi les composés répondant à la formule (II) suivante, et leurs sels d'addition : 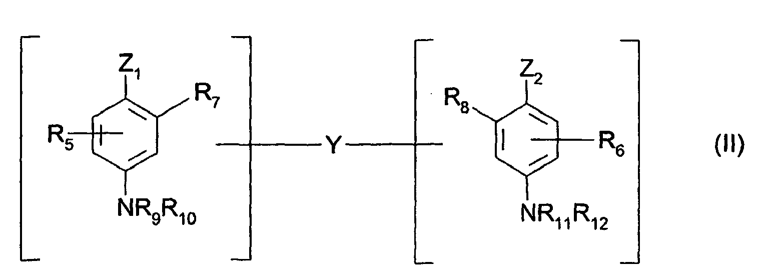 dans laquelle :
- Z₁ et Z₂, identiques ou différents, représentent un radical hydroxyle ou amino pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et / ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ; un atome d'halogène ; un radical alkyle en C₁-C₄ ; un radical monohydroxyalkyle en C₁-C₄ un radical polyhydroxyalkyle en C₂-C₄ ; un radical aminoalkyle en C₁-C₄ ou un bras de liaison Y ;
- R₇, R₈, R₉, R₁₀, R₁₁ et R₁₂, identiques ou différents, représentent un atome d'hydrogène ; un bras de liaison Y ; un radical alkyle en C₁-C₄ ou un radical monohydroxyalkyle en C₁-C₄ ;
étant entendu que les composés de formule (II) ne comportent qu'un seul bras de liaison Y par molécule.

6. Composition selon la revendication 5, dans laquelle les bases doubles de formule (II) sont choisies parmi le N,N'-bis-(β-hydroxyéthyl) N,N'-bis-(4'-aminophényl) 1,3-diamino propanol ; la N,N'-bis-(β-hydroxyéthyl) N,N'-bis-(4'-aminophényl) éthylènediamine ; la N,N'-bis-(4-aminophényl) tétraméthylènediamine ; la N,N'-bis-(β-hydroxyéthyl) N,N'-bis-(4-aminophényl) tétraméthylènediamine ; la N,N'-bis-(4-méthyl-aminophényl) tétraméthylènediamine ; la N,N'-bis-(éthyl) N,N'-bis-(4'-amino, 3'-méthylphényl) éthylènediamine ; le 1,8-bis-(2,5-diamino phénoxy)-3,6-dioxaoctane ; le 4-amino 6-[(5'-amino 2'-hydroxy 3'-méthyl phényl)méthyl] 2-méthyl phénol ; le bis-(5-amino 2-hydroxy phényl) méthane et leurs sels d'addition.

7. Compositions selon l'une quelconque des revendications 2 à 6, dans laquelle les para-aminophénols sont choisis parmi les composés répondant à la formule (III) suivante et leurs sels d'addition : 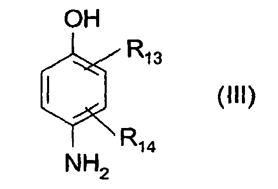 dans laquelle :
- R₁₃ représente un atome d'hydrogène ; un atome d'halogène ; un radical alkyle en C₁-C₄ ; un radical monohydroxyalkyle en C₁-C₄ ; un radical alcoxy(C₁-C₄)alkyle(C₁-C₄) ; un radical aminoalkyle en C₁-C₄ ou un radical hydroxyalkyl(C₁-C₄)aminoalkyle en C₁-C₄ ;
- R₁₄ représente un atome d'hydrogène ; un atome d'halogène ; un radical alkyle en C₁-C₄ ; un radical monohydroxyalkyle en C₁-C₄ un radical polyhydroxyalkyle en C₁-C₄ ; un radical aminoalkyle en C₁-C₄; un radical cyanoalkyle en C₁-C₄ ou un radical alcoxy(C₁-C₄)alkyle(C₁-C₄) ;
étant entendu qu'au moins un des radicaux R₁₃ ou R₁₄ représente un atome d'hydrogène.

8. Compositions selon la revendication 7, dans laquelle les para-aminophénols sont choisis parmi le para-aminophénol ; le 4-amino 3-méthyl phénol ; le 4-amino 3-fluoro phénol ; le 4-amino 3-hydroxyméthyl phénol ; le 4-amino 2-méthyl phénol ; le 4-amino 2-hydroxyméthyl phénol ; le 4-amino 2-méthoxyméthyl phénol ; le 4-amino 2-aminométhyl phénol ; le 4-amino 2-(β-hydroxyéthyl aminométhyl) phénol ; le 4-amino 2-fluoro phénol ; le 4-amino 2-chloro phénol ; le 4-amino 2,6-dichloro phénol et leurs sels d'addition.

9. Composition selon l'une quelconque des revendications 2 à 8, dans laquelle les ortho-aminophénols sont choisis parmi le 2-amino phénol ; le 2-amino 5-méthyl phénol ; le 2-amino 6-méthyl phénol ; le 5-acétamido 2-amino phénol et leurs sels d'addition.

10. Composition selon l'une quelconque des revendications 2 à 9, dans laquelle les bases hétérocycliques sont choisies parmi les dérivés pyridiniques, les dérivés pyrimidiniques, les dérivés pyrazoliques, les dérivés pyrazolo-pyrimidiniques et leurs sels d'addition.

11. Composition selon l'une quelconque des revendications précédentes, comprenant au moins un coupleur additionnel choisi parmi les méta-phénylènediamines, les méta-aminophénols autres que le 2-chloro 6-méthyl 3-amino phénol, les méta-diphénols, les coupleurs naphtaléniques, les coupleurs hétérocycliques autres que le 3-méthyl 1-phényl 5-pyrazolone ainsi que leurs sels d'addition.

12. Composition selon l'une quelconque des revendications précédentes, dans laquelle la quantité de chacune des bases d'oxydation est comprise entre 0,001 à 10 % en poids environ du poids total de la composition tinctoriale.

13. Composition selon l'une quelconque des revendications précédentes, dans laquelle la quantité de chacun des coupleurs est comprise entre 0,001 et 10 % en poids environ du poids total de la composition tinctoriale.

14. Composition selon l'une quelconque des revendications précédentes, dans laquelle le 3-méthyl 1-phényl 5-pyrazolone et / ou ses sels d'addition sont en quantité comprise entre 0,001 et 10 % en poids environ du poids total de la composition tinctoriale.

15. Composition selon l'une quelconque des revendications précédentes, dans laquelle le rapport entre le poids du 2-chloro 6-méthyl 3-amino phénol et / ou de ses sels d'addition et le poids du 3-méthyl 1-phényl 5-pyrazolone et / ou de ses sels d'addition est compris entre 1 et 100.

16. Composition selon l'une quelconque des revendications précédentes, comprenant un agent oxydant.

17. Composition selon la revendication 16, dans laquelle l'agent oxydant est choisi parmi le peroxyde d'hydrogène, le peroxyde d'urée, les bromates de métaux alcalines, les persels, les peracides et les enzymes oxydases.

18. Procédé de teinture d'oxydation des fibres kératiniques, dans lequel une composition telle que définie à l'une quelconque des revendications 1 à 15 est appliquée sur les fibres kératiniques, et que la couleur est révélée à l'aide d'un agent oxydant.

19. Procédé selon la revendication 18, dans lequel l'agent oxydant est choisi parmi le peroxyde d'hydrogène, le peroxyde d'urée, les bromates de métaux alcalins, les persels, les peracides et les enzymes oxydases.

20. Procédé selon la revendication 18 ou 19, dans lequel l'agent oxydant est mélangé au moment de l'emploi à la composition telle que définie à l'une quelconque des revendications 1 à 15.

21. Procédé selon la revendication 18 ou 19, dans lequel une composition comprenant l'agent oxydant est appliquée sur les fibres kératiniques simultanément ou séquentiellement à la composition telle que définie à l'une quelconque des revendications 1 à 15.

22. Dispositif à plusieurs compartiments, dans lequel un premier compartiment contient une composition tinctoriale telle que définie à l'une quelconque des revendications 1 à 15 et un deuxième compartiment contient une composition comprenant un agent oxydant.

23. Utilisation de la composition tinctoriale telle que définie à l'une quelconque des revendications 1 à 17 pour la teinture d'oxydation des fibres kératiniques.

24. Utilisation du 3-méthyl 1-phényl 5-pyrazolone et / ou de ses sels d'addition pour la stabilisation du 2-chloro 6-méthyl 3-amino phénol et / ou de ses sels d'addition dans les compositions.

## Claims

1. Composition for the oxidation dyeing of keratin fibres, comprising, in a suitable dyeing medium:
- at least one oxidation base;
- at least one coupler chosen from 2-chloro-6-methyl-3-aminophenol and the addition salts thereof; and
- 3-methyl-l-phenyl-5-pyrazolone and/or an addition salt thereof.

2. Composition according to Claim 1, in which the oxidation base(s) is (are) chosen from para-phenylenediamines, double bases, para-aminophenols, ortho-aminophenols and heterocyclic oxidation bases, and the addition salts thereof.

3. Composition according to Claim 2, in which the para-phenylenediamines are chosen from the compounds of formula (I) below, and the addition salts thereof: 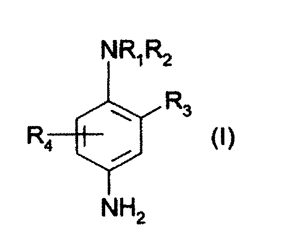 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical, a C₁-C₄ alkyl radical substituted with a nitrogenous group, a phenyl radical or a 4'-aminophenyl radical;
- R₂ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous group;
- R₁ and R₂ may form together, with the nitrogen atom to which they are attached, a 5- to 8-membered heterocycle optionally substituted with one or more groups chosen from C₁-C₄ alkyl, amino, C₁-C₄ hydroxyalkyl and trialkylammonium radicals;
- R₃ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino (C₁-C₄) alkoxy radical, a mesylamino (C₁-C₄) alkoxy radical or a carbamoylamino (C₁-C₄) alkoxy radical;
- R₄ represents a hydrogen or halogen atom or a C₁-C₄ alkyl radical.

4. Composition according to Claim 3, in which the para-phenylenediamines of formula (I) are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-2-methylaniline, 4-amino-N,N-bis (β-hydroxyethyl) -2-chloroaniline, 2-(β-hydroxyethyl)-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxy-ethyloxy)-para-phenylenediamine, 2-(β-acetylamino-ethyloxy)-para-phenylenediamine, N-(β-methoxyethyl)-para-phenylenediamine and the addition salts thereof.

5. Composition according to any one of Claims 2 to 4, in which the double bases are chosen from the compounds corresponding to formula (II) below, and the addition salts thereof: 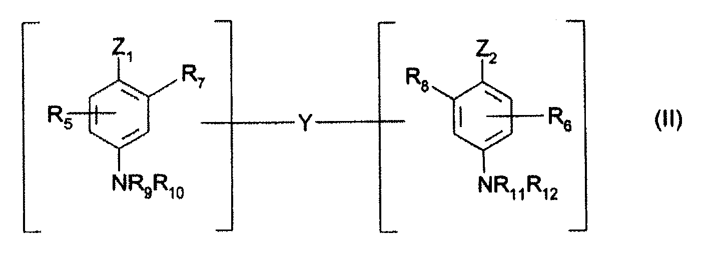 in which:
- Z₁ and Z₂, which may be identical or different, represent a hydroxyl or amino radical that may be substituted with a C₁-C₄ alkyl radical or with a linker arm Y;
- the linker arm Y represents a linear or branched alkylene chain containing from 1 to 14 carbon atoms, that may be interrupted by or terminated with one or more nitrogenous groups and/or one or more heteroatoms such as oxygen, sulfur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y, a C₁-C₄ alkyl radical or a C₁-C₄ monohydroxyalkyl radical;
it being understood that the compounds of formula (II) contain only one linker arm Y per molecule.

6. Composition according to Claim 5, in which the double bases of formula (II) are chosen from N, N' - bis (β-hydroxyethyl) -N,N' -bis (4'-aminophenyl) -1, 3-diaminopropanol, N,N'-bis(β-hydroxyethyl)-N,N'-bis(4'-aminophenyl)ethylenediamine, N,N'-bis(4-aminophenyl)-tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis-(ethyl)-N,N'-bis(4'-amino-3'-methylphenyl)ethylenediamine, 1,8-bis(2,5-diaminophenoxy)-3,6-dioxaoctane, 4-amino-6-[(5'-amino-2'-hydroxy-3'-methylphenyl)methyl]-2-methylphenol and bis(5-amino-2-hydroxyphenyl)methane, and the addition salts thereof.

7. Composition according to any one of Claims 2 to 6, in which the para-aminophenols are chosen from the compounds corresponding to formula (III) below, and the addition salts thereof: 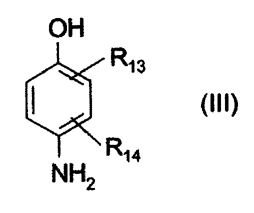 in which:
- R₁₃ represents a hydrogen atom, a halogen atom, or a C₁-C₄ alkyl, C₁-C₄ monohydroxyalkyl, (C₁-C₄) - alkoxy (C₁-C₄) alkyl, C₁-C₄ aminoalkyl or hydroxy (C₁-C₄) alkylamino (C₁-C₄) alkyl radical;
- R₁₄ represents a hydrogen atom, a halogen atom, or a C₁-C₄-alkyl, C₁-C₄ monohydroxyalkyl, C₂-C₄ polyhydroxyalkyl, C₁-C₄ aminoalkyl, C₁-C₄ cyanoalkyl or (C₁-C₄) alkoxy(C₁-C₄) alkyl radical;
it being understood that at least one of the radicals R₁₃ and R₁₄ represents a hydrogen atom.

8. Composition according to Claim 7, in which the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, 4-amino-2-fluorophenol, 4-amino-2-chlorophenol and 4-amino-2,6-dichlorophenol, and the addition salts thereof.

9. Composition according to any one of Claims 2 to 8, in which the ortho-aminophenols are chosen from 2-aminophenol, 2-amino-5-methylphenol, 2-amino-6-methylphenol and 5-acetamido-2-aminophenol, and the addition salts thereof.

10. Composition according to any one of Claims 2 to 9, in which the heterocyclic bases are chosen from pyridine derivatives, pyrimidine derivatives, pyrazole derivatives and pyrazolopyrimidine derivatives, and the addition salts thereof.

11. Composition according to any one of the preceding claims, comprising at least one additional coupler chosen from meta-phenylenediamines, meta-aminophenols other than 2-chloro-6-methyl-3-aminophenol, meta-diphenols, naphthalene-based couplers and heterocyclic couplers other than 3-methyl-1-phenyl-5-pyrazolone, and the addition salts thereof.

12. Composition according to any one of the preceding claims, in which the amount of each of the oxidation bases is between 0.001% and 10% by weight approximately relative to the total weight of the dye composition.

13. Composition according to any one of the preceding claims, in which the amount of each of the couplers is between 0.001% and 10% by weight approximately relative to the total weight of the dye composition.

14. Composition according to any one of the preceding claims, in which the 3-methyl-1-phenyl-5-pyrazolone and/or the addition salts thereof is/are in an amount of between 0.001% and 10% by weight approximately relative to the total weight of the dye composition.

15. Composition according to any one of the preceding claims, in which the ratio between the weight of the 2-chloro-6-methyl-3-aminophenol and/or the addition salts thereof and the weight of the 3-methyl-1-phenyl-5-pyrazolone and/or the addition salts thereof is between 1 and 100.

16. Composition according to any one of the preceding claims, comprising an oxidizing agent.

17. Composition according to Claim 16, in which the oxidizing agent is chosen from hydrogen peroxide, urea peroxide, alkali metal bromates, persalts, peracids and oxidase enzymes.

18. Process for the oxidation dyeing of keratin fibres, in which a composition as defined in any one of Claims 1 to 15 is applied to the keratin fibres, and the colour is developed using an oxidizing agent.

19. Process according to Claim 18, in which the oxidizing agent is chosen from hydrogen peroxide, urea peroxide, alkali metal bromates, persalts, peracids and oxidase enzymes.

20. Process according to Claim 18 or 19, in which the oxidizing agent is mixed at the time of use with the composition as defined in any one of Claims 1 to 15.

21. Process according to Claim 18 or 19, in which a composition comprising the oxidizing agent is applied to the keratin fibres simultaneously with or sequentially to the composition as defined in any one of Claims 1 to 15.

22. Multi-compartment device, in which a first compartment contains a dye composition as defined in any one of Claims 1 to 15 and a second compartment contains a composition comprising an oxidizing agent.

23. Use of the dye composition as defined in any one of Claims 1 to 17, for the oxidation dyeing of keratin fibres.

24. Use of 3-methyl-1-phenyl-5-pyrazolone and/or the addition salts thereof to stabilize 2-chloro-6-methyl-3-aminophenol and/or the addition salts thereof in the compositions.

## Patentansprüche

1. Zusammensetzung zum oxidativen Färben von Keratinfasern, die in einem zum Färben geeigneten Medium enthält:
- mindestens eine Oxidationsbase;
- mindestens einen Kuppler, der unter 2-Chlor-6-methyl-3-amino-phenol und seinen Additionssalzen ausgewählt ist; und
- 3-Methyl-1-phenyl-5-pyrazolon und/oder seine Additionssalze.

2. Zusammensetzung nach Anspruch 1, bei der die Oxidationsbase(n) unter den *p*-Phenylendiaminen, Doppelbasen, p-Aminophenolen, o-Aminophenolen und heterocyclischen Basen sowie deren Additionssalzen ausgewählt sind.

3. Zusammensetzung nach Anspruch 2, bei der die
*p*-Phenylendiamine unter den Verbindungen der folgenden Formel (I) und deren Additionssalzen ausgewählt sind: 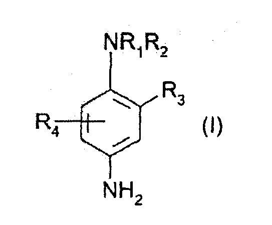 in der Formel:
- R₁ bedeutet ein Wasserstoffatom; C₁₋₄-Alkyl; C₁₋₄-Monohydroxyalkyl; C₂₋₄-Polyhydroxyalkyl; Alkoxy(C₁₋₄)-alkyl(C₁₋₄); eine mit einer Stickstoff-haltigen Gruppe substituierte C₁₋₄-Alkylgruppe; Phenyl oder 4'-Aminophenyl;
- R₂ bedeutet ein Wasserstoffatom, C₁₋₄-Alkyl; C₁₋₄-Monohydroxyalkyl; C₂₋₄-Polyhydroxyalkyl; Alkoxy(C₁₋₄)alkyl(C₁₋₄) oder eine mit einer Stickstoff-haltigen Gruppe substituierte C₁₋₄-Alkylgruppe;
- R₁ und R₂ können mit dem Stickstoffatom, an das sie gebunden sind, einen 5- bis 8-gliedrigen Heterocyclus bilden, der gegebenenfalls mit einer oder mehreren Gruppen substituiert ist, die unter C₁₋₄-Alkyl, Amino, C₁₋₄-Hydroxyalkyl und Trialkylammonium ausgewählt sind;
- R₃ bedeutet ein Wasserstoffatom; ein Halogenatom, wie Chlor, Brom, Iod oder Fluor; C₁₋₄-Alkyl; C₁₋₄-Monohydroxyalkyl; C₁₋₄-Hydroxyalkoxy; C₁₋₄-Acetylaminoalkoxy; C₁₋₄-Mesylaminoalkoxy oder C₁₋₄-Carbamoylaminoalkoxy;
- R₄ bedeutet ein Wasserstoffatom; ein Halogenatom oder C₁₋₄-Alkyl.

4. Zusammensetzung nach Anspruch 3, bei der die p-Phenylendiamine der Formel (I) ausgewählt sind unter:
*p*-Phenylendiamin; *p*-Toluylendiamin; 2-Chlor-*p*-phenylendiamin; 2,3-Dimethyl-*p*-phenylendiamin; 2,6-Dimethyl-p-phenylendiamin; 2,6-Diethyl-*p*-phenylendiamin; 2,5-Dimethyl-p-phenylendiamin; N,N-Dimethyl-*p*-phenylendiamin; N,N-Diethyl-p-phenylendiamin; N,N-Dipropyl-*p*-phenylendiamin; 4-Amino-N,N-diethyl-3-methylanilin; N,N-Bis(β-hydroxyethyl)-*p*-phenylendiamin; 4-N,N-Bis(β-hydroxyethyl)amino-2-methylanilin; 4-N,N-Bis(β-hydroxyethyl)amino-2-chloranilin; 2-β-Hydroxyethyl-*p-*phenylendiamin; 2-Fluor-*p*-phenylendiamin; 2-Isopropyl-p-phenylendiamin; N-(β-Hydroxypropyl)-*p*-phenylendiamin; 2-Hydroxymethyl-*p*-phenylendiamin; N,N-Dimethyl-3-methyl-p-phenylendiamin; N,N-(Ethyl, β-hydroxyethyl)-*p*-phenylendiamin; N-(β,γ-Dihydroxypropyl)-*p*-phenylendiamin; N-(4'-Aminophenyl)-*p*-phenylendiamin; N-Phenyl-*p*-phenylendiamin; 2-(β-Hydroxy-ethyloxy)-*p*-phenylendiamin; 2-(β-Acetylaminoethyloxy)-p-phenylendiamin; N-(β-Methoxyethyl)-*p*-phenylendiamin und deren Additionssalzen.

5. Zusammensetzung nach einem der Ansprüche 2 bis 4, bei der die Doppelbasen unter den Verbindungen der folgenden Formel (II) und deren Additionssalzen ausgewählt sind: 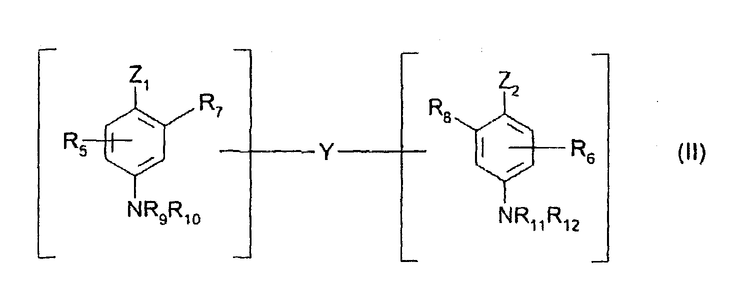 in der Formel:
- Z₁ und Z₂, die gleich oder verschieden sind, bedeuten eine Hydroxygruppe oder eine Aminogruppe, die mit einer C₁₋₄-Alkylgruppe oder einer Verbindungsgruppe Y substituiert sein können;
- die Verbindungsgruppe Y bedeutet eine geradkettige oder verzweigte Alkylengruppe mit 1 bis 14 Kohlenstoffatomen, die durch eine oder mehrere Stickstoff-haltige Gruppen und/oder durch ein oder mehrere Heteroatome, wie Sauerstoff, Schwefel oder Stickstoff, unterbrochen oder abgeschlossen und gegebenenfalls mit einer oder mehreren Hydroxygruppen oder C₁₋₆-Alkoxygruppen substituiert sein kann;
- R₅ und R₆ bedeuten ein Wasserstoffatom; ein Halogenatom; C₁₋₄-Alkyl; C₁₋₄-Monohydroxyalkyl; C₂₋₄-Polyhydroxyalkyl; C₁₋₄-Aminoalkyl oder eine Verbindungsgruppe Y;
- R₇, R₈, R₉, R₁₀, R₁₁ und R₁₂, die gleich oder verschieden sind, bedeuten ein Wasserstoffatom; eine Verbindungsgruppe Y; eine C₁₋₄-Alkylgruppe oder eine C₁₋₄-Monohydroxyalkylgruppe;
mit der Maßgabe, dass die Verbindungen der Formel (II) nur eine Verbindungsgruppe Y pro Molekül aufweisen.

6. Zusammensetzung nach Anspruch 5, bei der die Doppelbasen der Formel (II) unter N,N'-Bis(β-hydroxyethyl)-N,N'-bis(4'-aminophenyl)-1,3-diaminopropanol; N,N'-Bis(β-hydroxyethyl)-N,N'-bis(4'-aminophenyl)-ethylendiamin; N,N'-Bis(4-aminophenyl)-tetramethylendiamin; N,N'-Bis(β-hydroxyethyl)-N,N'-bis(4-aminophenyl)-tetramethylendiamin; N,N'-Bis(4-methylaminophenyl)-tetramethylendiamin; N,N'-Bis(ethyl)-N,N'-bis(4'-amino, 3'-methylphenyl)-ethylendiamin; 1,8-Bis(2,5-diaminophenoxy)-3,6-dioxaoctan; 4-Amino-6-[(5'-amino-2'-hydroxy-3'-methyl-phenyl)methyl]-2-methyl-phenol; Bis(5-amino-2-hydroxy-phenyl)-methan und deren Additionssalzen ausgewählt sind.

7. Zusammensetzung nach einem der Ansprüche 2 bis 6, bei der die p-Aminophenole unter den Verbindungen der folgenden Formel (III) und deren Additionssalzen ausgewählt sind: 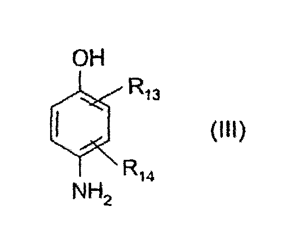 worin bedeuten:
- R₁₃ ein Wasserstoffatom; ein Halogenatom; C₁₋₄-Alkyl; C₁₋₄-Monohydroxyalkyl; Alkoxy(C₁₋₄)alkyl(C₁₋₄); C₁₋₄-Aminoalkyl oder Hydroxyalkyl(C₁₋₄)aminoalkyl(C₁₋₄);
- R₁₄ ein Wasserstoffatom; ein Halogenatom; C₁₋₄-Alkyl; C₁₋₄-Monohydroxyalkyl; C₂₋₄-Polyhydroxyalkyl; C₁₋₄-Aminoalkyl; C₁₋₄-Cyanoalkyl oder Alkoxy(C₁₋₄)alkyl(C₁₋₄);
mit der Maßgabe, dass mindestens eine der Gruppen R₁₃ oder R₁₄ ein Wasserstoffatom bedeutet.

8. Zusammensetzung nach Anspruch 7, bei der die p-Aminophenole unter p-Aminophenol; 4-Amino-3-methylphenol; 4-Amino-3-fluorphenol; 4-Amino-3-hydroxymethylphenol; 4-Amino-2-methylphenol; 4-Amino-2-hydroxymethylphenol; 4-Amino-2-methoxymethylphenol; 4-Amino-2-aminomethylphenol; 4-Amino-2-(β-hydroxyethylaminomethyl)phenol; 4-Amino-2-fluorphenol; 4-Amino-2-chlorphenol; 4-Amino-2,6-dichlorphenol und deren Additionssalzen ausgewählt sind.

9. Zusammensetzung nach einem der Ansprüche 2 bis 8, bei der die o-Aminophenole unter 2-Aminophenol; 2-Amino-5-methylphenol; 2-Amino-6-methylphenol; 5-Acetamido-2-aminophenol und deren Additionssalzen ausgewählt sind.

10. Zusammensetzung nach einem der Ansprüche 2 bis 9, bei der die heterocyclischen Basen unter den Pyridinderivaten, Pyrimidinderivate, Pyrazolderivaten, Pyrazolo-pyrimidinderivaten und deren Additionssalzen ausgewählt sind.

11. Zusammensetzung nach einem der vorhergehenden Ansprüche, die mindestens einen ergänzenden Kuppler enthält, der unter den *m*-Phenylendiaminen, *m*-Aminophenolen, die von 2-Chlor-6-methyl-3-amino-phenol verschieden sind, *m*-Dihydroxybenzolen, Naphthalinkupplern und heterocyclischen Kupplern, die von 3-Methyl-1-phenyl-5-pyrazolon verschieden sind, sowie deren Additionssalzen ausgewählt ist.

12. Zusammensetzung nach einem der vorhergehenden Ansprüche, bei der der Mengenanteil jeder Oxidationsbase im Bereich von etwa 0,001 bis 10 Gew.-% des Gesamtgewichts der Farbmittelzusammensetzung liegt.

13. Zusammensetzung nach einem der vorhergehenden Ansprüche, bei der der Mengenanteil jedes Kupplers im Bereich von etwa 0,001 bis 10 Gew.-% des Gesamtgewichts der Farbmittelzusammensetzung liegt.

14. Zusammensetzung nach einem der vorhergehenden Ansprüche, bei der das 3-Methyl-1-phenyl-5-pyrazolon und/oder seine Additionssalze in einem Mengenanteil von etwa 0,001 bis 10 Gew.-% des Gesamtgewichts der Farbmittelzusammensetzung enthalten sind.

15. Zusammensetzung nach einem der vorhergehenden Ansprüche, bei der das Gewichtsverhältnis des 2-Chlor-6-methyl-3-aminophenols und/oder seiner Additionssalze und des 3-Methyl-1-phenyl-5-pyrazolons und/oder seiner Additionssalze im Beeich von 1 bis 100 liegt.

16. Zusammensetzung nach einem der vorhergehenden Ansprüche, die ein Oxidationsmittel enthält.

17. Zusammensetzung nach Anspruch 16, bei der das Oxidationsmittel unter Wasserstoffperoxid, Harnstoffperoxid, Alkalimetallbromaten, Salzen von Persäuren, Persäuren und Oxidasen ausgewählt ist.

18. Verfahren zum oxidativen Färben von Keratinfasern, bei dem eine Zusammensetzung, wie sie in einem der Ansprüche 1 bis 15 definiert ist, auf die Keratinfasern aufgebracht wird und die Farbe mit Hilfe eines Oxidationsmittels entwickelt wird.

19. Verfahren nach Anspruch 18, bei dem das Oxidationsmittel unter Wasserstoffperoxid, Harnstoffperoxid, Alkalimetallbromaten, Salzen von Persäuren, Persäuren und Oxidasen ausgewählt ist.

20. Verfahren nach Anspruch 18 oder 19, bei dem das Oxidationsmittel bei der Anwendung mit der Zusammensetzung nach einem der Ansprüche 1 bis 15 vermischt wird.

21. Verfahren nach Anspruch 18 oder 19, bei dem eine Zusammensetzung, die das Oxidationsmittel enthält, gleichzeitig mit oder nach der Zusammensetzung nach einem der Ansprüche 1 bis 15 auf die Keratinfasern aufgebracht wird.

22. Vorrichtung mit mehreren Abteilungen, wobei eine erste Abteilung eine Farbmittelzusammensetzung nach einem der Ansprüche 1 bis 15 enthält und eine zweite Abteilung eine Zusammensetzung mit einem Oxidationsmittel enthält.

23. Verwendung der Farbmittelzusammensetzung nach einem der Ansprüche 1 bis 17 zum oxidativen Färben von Keratinfasern.

24. Verwendung von 3-Methyl-1-phenyl-5-pyrazolon und/oder seiner Additionssalze zur Stabilisierung von 2-Chlor-6-methyl-3-aminophenol und/ oder seiner Additionssalze in Zusammensetzungen.
